# EUROPEAN PATENT SPECIFICATION

(11) **EP 0 739 350 B1**
(45) Date of publication and mention of the grant of the patent: **27.02.2008**
(21) Application number: 95907381.8
(22) Date of filing: 06.01.1995
(51) Int. Cl.: C07H 21/04, C12N 15/19, C12N 15/63, C07K 14/52, C07K 16/24

(54) **LIGAND THAT BINDS FAS ANTIGEN**
FAS-ANTIGENBINDENDER LIGAND
LIGAND FIXANT L'ANTIGENE FAS

(30) Priority: 07.01.1994 US 179138; 01.02.1994 US 190559
(43) Date of publication of application: 30.10.1996
(73) Proprietor: MOCHIDA PHARMACEUTICAL CO., LTD., Shinjuku-ku, Tokyo 160-0004 (JP); OSAKA BIOSCIENCE INSTITUTE, Suita-shi, Osaka 565 (JP)
(72) Inventor: GOODWIN, Raymond, G., Seattle, WA 98119 (US)
(74) Representative: Zimmer, Franz-Josef
(86) International application number: PCT/US1995/000362
(87) International publication number: WO 1995/018819

(56) References cited:
- EP-A- 0 510 691
- EP-A- 0 675 200
- WO-A-91/10448
- TAKAHASHI T: "Human Fas ligand: gene structure, chromosomal location and species specificty" INTERNATIONAL IMMUNOLOGY, vol. 6, no. 10, October 1994, pages 1567-1574, XP002072758
- ARTHRITIS AND RHEUMATISM, Volume 36(9), issued 1993, Y. JIAN et al., "The Fas-Ligand and Apoptosis in LPR and GLD Mice", page s52, (Abstract No. 80).
- ARTHRITIS AND RHEUMATISM, Volume 36, No. 9, issued 1993, T. ZHOU et al., "FAS+ COS Cells and Anti-FAS Antibody for In Vivo Localization of FAS/FAS Ligand Binding and Apoptosis Sites in Autoimmune LPR and GLD Mice", page s52, (Abstract No. 81).
- CELL, Volume 75, issued 17 December 1993, T. SUDA et al., "Molecular Cloning and Expression of the FAS Ligand, a Novel Member of the Tumor Necrosis Factor Family", pages 1169-1178.
- NATURE, Volume 356, issued 26 March 1992, R. WATANABE-FUKUNAGA et al., "Lymphoproliferation Disorder in Mice Explained by Defects in Fas Antigen that Mediates Apoptosis", pages 314-317.
- J. BIOL. CHEM., Volume 267, No. 15, issued 25 May 1992, A. OEHM et al., "Purification and Molecular Cloning of the APO-1 Cell Surface Antigen, a Member of the Tumor Necrosis Factor/Nerve Growth Factor Receptor Superfamily", pages 10709-10715.
- J. IMMUNOL., Volume 148, No. 4, issued 15 February 1992, R. WATANABE-FUKUNAGA et al., "The cDNA Structure, Expression, and Chromosomal Assignment of the Mouse Fas Antigen", pages 1274-1279.
- J. EXP. MED., Volume 172, issued November 1990, R.D. ALLEN et al., "Differences Defined by Bone Marrow Transplantation Suggest that 1pr and gld are Mutations of Genes Encoding and Interacting Pair of Molecules", pages 1367-1375.
- CURRENT PROTOCOLS IN IMMUNOLOGY, Supplement 4, issued 1992, D. HOLLENBAUGH et al., "Construction of Immunoglobulin Fusion Proteins", pages 10.19.1-10.19.11.
- PROC. NAT. ACAD. SCI. USA, Volume 84, issued December 1987, A. ARUFFO et al., "Molecular Cloning of a CD28 cDNA by a High-Efficiency COS Cell Expression System", pages 8573-8577.

## Description

The programmed cell death known as apoptosis occurs in embryogenesis, metamorphosis, endocrine-dependent tissue atrophy, normal tissue turnover, and death of immune thymocytes (induced through their antigen-receptor complex or by glucocorticoids) (Itoh et al., Cell 66:233, 1991). During maturation of T-cells in the thymus, T-cells that recognize self-antigens are destroyed through the apoptotic process, whereas others are positively selected. The possibility that some T-cells recognizing certain self epitopes (e.g., inefficiently processed and presented antigenic determinants of a given self protein) escape this elimination process and subsequently may play a role in autoimmune diseases has been suggested (Gammon et al., Immunology Today 12:193, 1991).

A cell surface antigen known as Fas has been reported to mediate apoptosis and is believed to play a role in clonal deletion of self-reactive T-cells (Itoh et al., Cell 66:233, 1991; Watanabe-Fukunage et al., Nature 356:314, 1992). Fas is a 45kDa molecule expressed on activated T cells, B cells, and neutrophils (Itoh, *supra*). High levels of Fas mRNA have been detected in thymus, liver, heart, lung and ovary of mice (Watanabe-Fukunaga et al., J. Immunol., 148:1274, 1992). Cross-linking a specific monoclonal antibody to Fas has been reported to induce various cell lines to undergo apoptosis (Yonehara et al., J. Exp. Med., 169:1747, 1989; Trauth et al., Science, 245:301, 1989). However, under certain conditions, binding of a specific monoclonal antibody to Fas can be stimulatory for freshly isolated T cells (Alderson et al., J. Exp. Med. 178:2231, 1993).

Cloning of cDNA encoding murine and human Fas antigen has been reported by Watanage-Fukunage et al. (J. Immunol.*, supra*) and Itoh et al., *supra,* respectively. The amino acid sequences encoded by the cloned cDNAs indicate that both murine and human Fas antigens are transmembrane proteins exhibiting structural homology with members of the nerve growth factor receptor/tumor necrosis factor receptor (NGFR/TNFR) family of cell surface receptors.

Mice carrying the autosomal recessive mutation known as the lymphoproliferation (*lpr*) mutation have defects in the Fas antigen gene and do not express normal functional Fas protein capable of transducing the apoptotic signal (Watanabe-Fukunage et al., Nature 356:314,1992). Mice carrying an *lpr* mutation develop lymphoproliferative disorders characterized by the accumulation of CD4 CD8- thymocytes in lymph nodes and the spleen, hypergammaglobulinaemia, autoantibody production, rheumatoid factor, arthritis and glomerulonephritis. (Watanabe-Fukunage et al., Nature*, supra*).

A clinical syndrome indistinguishable from that found in *lpr* mice is exhibited by mice carrying a mutant gene known as the generalized lymphoproliferative disease (*gld*) mutation (Watanabe-Fukunage et al., Nature*, supra*). The *gld* and *lpr* mutations involve different genes which map to different chromosomes (Seldin et al., J. Exp. Med 167:688, 1988; Watson et al., Mammalian Genome 2:158,1992-. Watanabe-Fukunage et al., Biochem. Genet. 29:325, 1991; and Watanabe-Fukunage et al., J. Immunol*, supra*).

Investigation into the existence and identity of molecule(s) that interact with the Fas antigen is desirable in order to provide further insight into the development of self-tolerance by the immune system, and provide a means for studying the etiology of autoimmune diseases. cDNA encoding a rat protein that binds Fas has been cloned (Suda et al., Cell, 75:1169, 1993). A human Fas ligand would be preferred for such uses as research involving the human immune system and development of diagnostic or therapeutic agents for use in humans. Prior to the present invention, however, no human protein that binds to the Fas antigen was known.

### SUMMARY OF THE INVENTION

The present invention provides novel Fas ligand (Fas-L) proteins, as well as isolated DNA encoding the Fas-L proteins, expression vectors comprising the isolated DNA, and a method for producing Fas-L by cultivating host cells containing the expression vectors under conditions appropriate for expression of the Fas-L protein. Fas-L is a protein that binds to the antigen known as Fas (a cell surface protein). In particular embodiments, the Fas-L is a human or murine protein. Antibodies directed against the Fas-L protein or an immunogenic fragment thereof are also provided. Among the uses of the antibodies is blocking the binding of Fas-L to Fas. Biological activities mediated by such binding thus may be inhibited.

**The present invention provides the following aspects:**
1. An isolated DNA encoding a human Fas-L polypeptide, wherein said Fas-L polypeptide is capable of binding Fas and is characterized by an N-terminal amino acid sequence MQQPFNYPYPQI and wherein said Fas-L polypeptide comprises the amino acid sequence of residues 1-281 of SEQ ID NO:2.
2. An isolated DNA according to claim 1, wherein said DNA comprises the nucleotide sequence of nucleotides 93-938 of SEQ ID NO:1.
3. An isolated DNA according to claim 1, wherein said DNA comprises the coding region of the cDNA insert of the recombinant vector deposited in strain ATCC 69527.
4. An isolated DNA encoding a soluble human Fas-L polypeptide, wherein said Fas-L polypeptide comprises the amino acid sequence of residues 106-281 of SEQ ID NO:2.
5. An isolated DNA according to claim 4, wherein said DNA comprises the nucleotide sequence of nucleotides 408-938 of SEQ ID NO: 1.
6. An expression vector comprising a DNA as defined in any one of claims 1 to 5.
7. A process for preparing a Fas-L polypeptide, comprising culturing a host cell transformed with a vector as defined in claim 6 under conditions promoting expression of Fas-L, and recovering the Fas-L polypeptide from the culture.
8. A purified human Fas-L protein characterized by an N-terminal amino acid sequence MQQPFNYPYPQI, wherein said Fas-L protein is capable of binding Fas, and wherein said protein comprises the amino acid sequence of residues 1-281 of SEQ ID NO:2.
9. A soluble human Fas-L protein comprising the amino acid sequence of residues 106-281 of SEQ ID NO:2.
10. A human Fas-L protein of claim 8 encoded by the cDNA insert of the recombinant vector deposited in strain ATCC 69527.
11. An oligomer of Fas-L polypeptides comprising a polypeptide of any one of claims 8 to 10.
12. An oligomer according to claim 11 in the form of dimer or trimer of the polypeptide of any one of claims 8 to 10.
13. An oligomer according to claim 11 or 12, which is formed by disulfide bonds between cysteine residues on different Fas-L polypeptides or expressed as fusion polymers with or without spacer amino acid linking groups.
14. An oligomer according to claim 11, wherein said oligomer is a dimer and is created by fusing Fas-L to the Fc region of an antibody.
15. An isolated nucleic acid molecule comprising at least about 14 nucleotides of SEQ ID NO:1, or the DNA or RNA complement thereof.
16. A human Fas-L polypeptide according to any of claims 8 to 10 for use in medicine.
17. An antibody, immunoreactive with human Fas-L according to any of claims 8 to 10 for use in medicine.
18. Use of a human Fas-L polypeptide according to any of claims 8 to 10 or DNA encoding a human Fas-L polypeptide according to any of claims 11 to 14 in the preparation of an agent for the treatment of an autoimmune disease, graft versus host disease, or a disease mediated by the interaction of Fas-L and Fas selected from systemic lupus erythematosus (SLE), rheumatoid arthritis, lyme disease, idiopathic CD4⁺ T lymphocytopenia or the effects of human immunodeficiency virus (HIV) infection.
19. The use according to claim 18 in which the autoimmune disease is systemic lupus erythematosus (SLE), immunoblastive lymphadenopathy (IBL), angioimmunoblastic lymphadenopathy (AIL), lymphogranulomatosis X (LgX), rheumatoid arthritis, diabetes, multiple sclerosis or allergies.
20. Use of an antibody that is immunoreactive with human Fas-L according to any of claims 8 to 10 and that inhibits binding of a Fas-L polypeptide to Fas in the preparation of an agent for the treatment of a disease mediated by the interaction of Fas-L and Fas selected from systemic lupus erythematosus (SLE), rheumatoid arthritis, lyme disease, idiopathic CD4⁺ T lymphocytopenia or the effects of human immunodeficiency virus (HIV) infection.
21. A pharmaceutical composition comprising a human Fas-L polypeptide according to any of claims 8 to 10, and optionally a pharmaceutically acceptable excipient, diluent or carrier.
22. A pharmaceutical composition comprising an antibody immunoreactive with a human Fas-L polypeptide according to any of claims 8 to 10, and optionally a pharmaceutically acceptable excipient, diluent or carrier.

### DETAILED DESCRIPTION OF THE INVENTION

DNA encoding a novel human polypeptide that can act as a ligand for the cell surface antigen known as Fas has been isolated in accordance with the present invention. Murine Fas-L DNA is provided as well. Also provided are expression vectors comprising the Fas ligand (Fas-L) DNA and methods for producing recombinant Fas-L polypeptides by cultivating host cells containing the expression vectors under conditions appropriate for expression of Fas-L, and recovering the expressed Fas-L. Substantially homogeneous purified Fas-L protein is also encompassed by the present invention.

The present invention also provides human or murine Fas-L or antigenic fragments thereof that can act as immunogens to generate antibodies specific to the Fas-L immunogens. Monoclonal antibodies specific for Fas-L or antigenic fragments thereof thus can be prepared.

The novel protein disclosed herein is a ligand for Fas, a cell surface protein that is a member of the TNF/NGF receptor superfamily. Fas is believed to play a role in clonal deletion of self-reactive T-cells. Mice carrying mutations that cause production of defective Fas antigen are afflicted with an autoimmune syndrome. One use of the Fas ligand of the present invention is as a research tool for studying autoimmune disorders and investigating the roles that Fas and Fas-L may play in inducing self-tolerance. The Fas-L polypeptides of the present invention also may be employed in *in vitro* assays for detection of Fas or Fas-L or the interactions thereof.

The Fas-L protein may be employed as a protein purification reagent. Fas-L attached to a solid support material is useful for purifying Fas protein by affinity chromatography. Fas-L also finds use in monitoring the biological activity of Fas proteins, as described in more detail below.

Isolation of a cDNA encoding human Fas-L is described in example 2 below. *E. coli* strain DH5α transformed with the cloning vector pBLUESCRIPT^{®}SK containing this Fas-L cDNA was deposited with the American Type Culture Collection on January 5, 1994, and assigned accession no. 69527. The deposit was made under the terms of the Budapest Treaty.

The nucleotide sequence of the human Fas-L DNA isolated in example 2 is presented in SEQ ID NO: 1. The amino acid sequence encoded thereby is presented in SEQ ID NO:2. This human Fas-L protein comprises an N-terminal cytoplasmic domain (amino acids 1-80), a transmembrane region (amino acids 81-105), and an extracellular domain (amino acids 106-281). The extracellular domain contains the receptor-binding region.

As used herein, the term "Fas-L" includes membrane-bound proteins (comprising a cytoplasmic domain, a transmembrane region, and an extracellular domain) as well as truncated proteins that retain the Fas-binding property. In one embodiment, soluble Fas-L polypeptides comprise all or part of the extracellular domain of a Fas-L protein, but lack the transmembrane region that would cause retention of the polypeptide on a cell membrane. Advantageously, a heterologous signal peptide is fused to the N-terminus such that the soluble Fas-L is secreted upon expression. The soluble Fas-L polypeptides that may be employed retain the ability to bind the Fas antigen. Soluble Fas-L may also include part of the transmembrane region or part of the cytoplasmic domain or other sequences, provided that the soluble Fas-L protein is capable of being secreted.

Soluble Fas-L may be identified (and distinguished from its non-soluble membrane-bound counterparts) by separating intact cells which express the desired protein from the culture medium, e.g., by centrifugation, and assaying the medium (supernatant) for the presence of the desired protein. The culture medium may be assayed using procedures which are similar or identical to those described in the examples below. The presence of Fas-L in the medium indicates that the protein was secreted from the cells and thus is a soluble form of the desired protein. Soluble Fas-L may be a naturally-occurring form of this protein.

The use of soluble forms of Fas-L is advantageous for certain applications. Purification of the proteins from recombinant host cells is facilitated, since the soluble proteins are secreted from the cells. Further, soluble proteins are generally more suitable for intravenous administration.

Truncated Fas-L, including soluble polypeptides, may be prepared by any of a number of conventional techniques. In the case of recombinant proteins, a DNA fragment encoding a desired fragment may be subcloned into an expression vector. Alternatively, a desired DNA sequence may be chemically synthesized using known techniques. DNA fragments also may be produced by restriction endonuclease digestion of a full length cloned DNA sequence, and isolated by electrophoresis on agarose gels. Linkers containing restriction endonuclease cleavage site(s) may be employed to insert the desired DNA fragment into an expression vector, or the fragment may be digested at cleavage sites naturally present therein. The well known polymerase chain reaction procedure also may be employed to isolate a DNA sequence encoding a desired protein fragment.

In another approach, enzymatic treatment (e.g., using Bal 31 exonuclease) may be employed to delete terminal nucleotides from a DNA fragment to obtain a fragment having a particular desired terminus. Among the commercially available linkers are those that can be ligated to the blunt ends produced by Bal 31 digestion, and which contain restriction endonuclease cleavage site(s). Alternatively, oligonucleotides that reconstruct the N- or C-terminus of a DNA fragment to a desired point may be synthesized. The oligonucleotide may contain a restriction endonuclease cleavage site upstream of the desired coding sequence and position an initiation codon (ATG) at the N-terminus of the coding sequence.

The Fas-L DNA of the present invention includes cDNA, chemically synthesized DNA, DNA isolated by PCR, genomic DNA, and combinations thereof. Genomic Fas-L DNA may be isolated by hybridization to the Fas-L cDNA disclosed herein using standard techniques. RNA transcribed from the Fas-L DNA is also encompassed by the present invention.

Certain embodiments of the present invention provide isolated DNA comprising a nucleotide sequence selected from the group consisting of nucleotides 93-938 (coding region) or 408-938 (encoding the extracellular domain) of SEQ ID NO: 1. DNA encoding biologically active fragments of the protein of SEQ ID NO:2 are also provided.

Also provided herein are purified Fas-L polypeptides, both recombinant and nonrecombinant. Variants and derivatives of native Fas-L proteins that retain the desired biological activity are also within the scope of the present invention. Fas-L variants may be obtained by mutations of nucleotide sequences coding for native Fas-L polypeptides, for example. A Fas-L variant, as referred to herein, is a polypeptide substantially homologous to a native Fas-L, but which has an amino acid sequence different from that of native Fas-L because of one or a plurality of deletions, insertions or substitutions. Fas-L-encoding DNA sequences of the present invention encompass sequences that comprise one or more additions, deletions, or substitutions of nucleotides when compared to a native Fas-L DNA sequence, but that encode a Fas-L protein that is essentially bioequivalent to a native Fas-L protein.

The variant amino acid or DNA sequence preferably is at least 90% identical to a native Fas-L sequence. The degree of homology (percent identity) between a native and a mutant sequence may be determined, for example, by comparing the two sequences using the GAP computer program, version 6.0, described by Devereux et al. (Nucl. Acids Res. 12:387, 1984) and available from the University of Wisconsin Genetics Computer Group (UWGCG). The GAP program utilizes the alignment method of Needleman and Wunsch (J. Mol. Biol. 48:443, 1970), as revised by Smith and Waterman (Adv. Appl. Math 2:482, 1981). Briefly, the GAP program defines similarity as the number of aligned symbols (i.e., nucleotides or amino acids) which are similar, divided by the total number of symbols in the shorter of the two sequences. The preferred default parameters for the GAP program include: (1) a unary comparison matrix (containing a value of 1 for identities and 0 for non-identities) for nucleotides, and the weighted comparison matrix of Gribskov and Burgess, Nucl. Acids Res. 14:6745, 1986, as described by Schwartz and Dayhoff, eds., Atlas of Protein Sequence and Structure, National Biomedical Research Foundation, pp. 353-358, 1979; (2) a penalty of 3.0 for each gap and an additional 0.10 penalty for each symbol in each gap; and (3) no penalty for end gaps.

Alterations of the native amino acid sequence may be accomplished by any of a number of known techniques. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion.

Alternatively, oligonucleotide-directed site-specific mutagenesis procedures can be employed to provide an altered gene having particular codons altered according to the substitution, deletion, or insertion required. Exemplary methods of making the alterations set forth above are disclosed by Walder et al. (Gene 42:133, 1986); Bauer et al. (Gene 37:73, 1985); Craik (BioTechniques, January 1985, 12-19); Smith et al. (Genetic Engineering: Principles and Methods, Plenum Press, 1981); and U.S. Patent Nos. 4,518,584 and 4,737,462, which are incorporated by reference herein.

Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known.

Fas-L also may be modified to create Fas-L derivatives by forming covalent or aggregative conjugates with other chemical moieties, such as glycosyl groups, lipids, phosphate, acetyl groups and the like. Covalent derivatives of Fas-L may be prepared by linking the chemical moieties to functional groups on Fas-L amino acid side chains or at the N-terminus or C-terminus of a Fas-L polypeptide or the extracellular domain thereof. Other derivatives of Fas-L within the scope of this invention include covalent or aggregative conjugates of Fas-L or its fragments with other proteins or polypeptides, such as by synthesis in recombinant culture as N-terminal or C-terminal fusions. For example, the conjugate may comprise a signal or leader polypeptide sequence (e.g. the α-factor leader of *Saccharomyces*) at the N-terminus of a Fas-L polypeptide. The signal or leader peptide co-translationally or post-translationally directs transfer of the conjugate from its side of synthesis to a site inside or outside of the cell membrane or cell wall.

Fas-L polypeptide fusions can comprise peptides added to facilitate purification and identification of Fas-L. Such peptides include, for example, poly-His or the antigenic identification peptides described in U.S. Patent No. 5,011,912 and in Hopp et al., BiolTechnology 6:1204, 1988. One such peptide is the FLAG® peptide, Asp-Tyr-Lys-Asp-Asp-Asp-Asp-Lys (DYKDDDDK) (SEQ ID NO:3), which is highly antigenic and provides an epitope reversibly bound by a specific monoclonal antibody enabling rapid assay and facile purification of expressed recombinant protein. This sequence is also specifically cleaved by bovine mucosal enterokinase at the residue immediately following the Asp-Lys pairing. Fusion proteins capped with this peptide may also be resistant to intracellular degradation in *E. coli.* A murine hybridoma designated 4E11 produces a monoclonal antibody that binds the peptide DYKDDDDK (SEQ ID NO:3) in the presence of certain divalent metal cations (as described in U.S. Patent 5,011,912) and has been deposited with the American Type Culture Collection under accession no HB 9259. Monoclonal antibodies that bind the Flags^{®} peptide are available from Eastman Kodak Co., Scientific Imaging Systems Division, New Haven, Connecticut.

The present invention further includes Fas-L polypeptides with or without associated native-pattern glycosylation. Fas-L expressed in yeast or mammalian expression systems (e.g., COS-7 cells) may be similar to or significantly different from a native Fas-L polypeptide in molecular weight and glycosylation pattern, depending upon the choice of expression system. Expression of Fas-L polypeptides in bacterial expression systems, such as *E. coli,* provides non-glycosylated molecules.

DNA constructs that encode various additions or substitutions of amino acid residues or sequences, or deletions of terminal or internal residues or sequences not needed for biological activity or binding can be prepared. For example, N-glycosylation sites in the Fas-L extracellular domain can be modified to preclude glycosylation while allowing expression of a homogeneous, reduced carbohydrate analog using yeast expression systems. N-glycosylation sites in eukaryotic polypeptides are characterized by an amino acid triplet Asn-X-Y, wherein X is any amino acid except Pro and Y is Ser or Thr. Such sites are found in human Fas-L at amino acids 76-78,184-186, 250-252, and 260-262 of SEQ ID NO: 2. Appropriate modifications to the nucleotide sequence encoding this triplet will result in substitutions, additions or deletions that prevent attachment of carbohydrate residues at the Asn side chain. Known procedures for inactivating N-glycosylation sites in proteins include those described in U.S. Patent 5,071,972 and EP 276,846.

In another example, sequences encoding Cys residues that are not essential for biological activity can be altered to cause the Cys residues to be deleted or replaced with other amino acids, preventing formation of incorrect intramolecular disulfide bridges upon renaturation. Other variants are prepared by modification of adjacent dibasic amino acid residues to enhance expression in yeast systems in which KEX2 protease activity is present. EP 212,914 discloses the use of site-specific mutagenesis to inactivate KEX2 protease processing sites in a protein. KEX2 protease processing sites are inactivated by deleting, adding or substituting residues to alter Arg-Arg, Arg-Lys, and Lys-Arg pairs to eliminate the occurrence of these adjacent basic residues. Such KEX2 protease processing sites are found at residues 38-39, 43-44, 73-74, and 144-145 of SEQ ID NO:2. Lys-Lys pairings are considerably less susceptible to KEX2 cleavage, and conversion of Arg-Lys or Lys-Arg to Lys-Lys represents a conservative and preferred approach to inactivating KEX2 sites.

Naturally occurring Fas-L variants are also encompassed by the present invention. Examples of such variants are proteins that result from alternative mRNA splicing events (since Fas-L is encoded by a multi-exon gene) or from proteolytic cleavage of the Fas-L protein, wherein the Fas-binding property is retained. Alternative splicing of mRNA may yield a truncated but biologically active Fas-L protein, such as a naturally occurring soluble form of the protein, for example. Variations attributable to proteolysis include, for example, differences in the N- or C-termini upon expression in different types of host cells, due to proteolytic removal of one or more terminal amino acids from the Fas-L protein. Fas-L proteins having amino acid sequences that are truncated at the N- or C- terminus by from one to five amino acids are encompassed by the present invention. In addition, proteolytic cleavage may release a soluble form of Fas-L from a membrane-bound form of the protein.

Due to degeneracy of the genetic code, two DNA sequences may differ, yet encode the same amino acid sequence. The present invention thus provides isolated DNA sequences encoding biologically active Fas-L, selected from DNA comprising the coding region of a native human or murine Fas-L cDNA, or fragments thereof, and DNA which is degenerate as a result of the genetic code to the native Fas-L DNA sequence. One embodiment of the present invention provides DNA comprising the coding region of the nucleotide sequence of SEQ ID NO:1, as well as DNA sequences degenerate thereto.

Variants possessing the requisite ability to bind Fas may be identified by any suitable assay. Biological activity of Fas-L may be determined, for example, by competition for binding to the ligand binding domain of Fas (i.e. competitive binding assays).

### Assays

One type of a competitive binding assay for Fas-L polypeptide uses a radiolabeled, soluble human Fas-L and intact cells expressing cell surface Fas. Instead of intact cells, one could substitute soluble Fas (such as a Fas/Fc fusion protein) bound to a solid phase through a Protein A or Protein G interaction with the Fc region of the fusion protein. Another type of competitive binding assay utilizes radiolabeled soluble Fas such as a Fas/Fc fusion protein, and intact cells expressing Fas-L. Alternatively, soluble Fas-L could be bound to a solid phase.

Competitive binding assays can be performed using standard methodology. Qualitative results can be obtained by competitive autoradiographic plate binding assays, or Scatchard plots may be utilized to generate quantitative results.

Competitive binding assays with intact cells expressing Fas can be performed by two methods. In a first method, cells expressing endogenous cell surface Fas are grown either in suspension or by adherence to tissue culture plates. Adherent cells can be removed by treatment with 5 mM EDTA treatment for ten minutes at 37° C. In a second method, transfected cells expressing membrane-bound recombinant Fas can be used. COS cells or another mammalian cell can be transfected with human Fas cDNA in an appropriate vector to express full length Fas with an extracellular region.

Alternatively, soluble Fas can be bound to a solid phase such as a column chromatography matrix or a similar substrate. Binding to a solid phase can be accomplished, for example, by preparing a Fas/Fc fusion protein and binding it to a protein A or protein G-containing matrix.

Another means to measure the biological activity of Fas-L (including variants) is to utilize conjugated, soluble Fas (for example, ¹²⁵I-Fas/Fc) in competition assays similar to those described above. In this case, however, intact cells expressing Fas-L, or soluble Fas-L bound to a solid substrate, are used to measure competition for binding of conjugated, soluble Fas to Fas-L by a sample containing a putative Fas-L variant.

### Oligomers

The present invention encompasses Fas-L polypeptides in the form of oligomers, such as dimers or trimers. Oligomers may be formed by disulfide bonds between cysteine residues on different Fas-L polypeptides. In one embodiment of the invention, a Fas-L dimer is created by fusing Fas-L to the Fc region of an antibody (IgG 1). The term "Fc polypeptide" includes native and mutein forms, as well as truncated Fc polypeptides containing the hinge region that promotes dimerization. The Fc polypeptide preferably is fused to a soluble Fas-L (comprising only the extracellular domain). Preparation of fusion proteins comprising heterologous polypeptides fused to various portions of antibody-derived polypeptides (including the Fc domain) has been described, e.g., by Ashkenazi et al. (PNAS USA 88:10535, 1991) and Byrn et al. (Nature 344:667, 1990), hereby incorporated by reference.

A gene fusion encoding the Fas-L/Fc fusion protein is inserted into an appropriate expression vector. The Fas-L/Fc fusion proteins are allowed to assemble much like antibody molecules, whereupon interchain disulfide bonds form between Fc polypeptides, yielding divalent Fas-L. In other embodiments, Fas-L may be substituted for the variable portion of an antibody heavy or light chain. If fusion proteins are made with both heavy and light chains of an antibody, it is possible to form a Fas-L oligomer with as many as four Fas-L extracellular regions. Alternatively, one can link two soluble Fas-L polypeptides with a peptide linker such as those described in United States Patent 5,073,627 (hereby incorporated by reference).

The present invention provides oligomers of Fas-L extracellular domains or fragments thereof, linked by disulfide interactions, or expressed as fusion polymers with or without spacer amino acid linking groups. For example, a dimer Fas-L molecule can be linked by an IgG Fc region linking group.

### Expression Systems

The present invention provides recombinant expression vectors for expression of Fas-L, and host cells transformed with the expression vectors. Any suitable expression system may be employed. The vectors include a DNA encoding a Fas-L polypeptide, operably linked to suitable transcriptional or translational regulatory nucleotide sequences, such as those derived from a mammalian, microbial, viral, or insect gene. Examples of regulatory sequences include transcriptional promoters, operators, or enhancers, an mRNA ribosomal binding site, and appropriate sequences which control transcription and translation initiation and termination. Nucleotide sequences are operably linked when the regulatory sequence functionally relates to the Fas-L DNA sequence. Thus, a promoter nucleotide sequence is operably linked to a Fas-L DNA sequence if the promoter nucleotide sequence controls the transcription of the Fas-L DNA sequence. The ability to replicate in the desired host cells, usually conferred by an origin of replication, and a selection gene by which transformants are identified, may additionally be incorporated into the expression vector.

In addition, sequences encoding appropriate signal peptides that are not native to the Fas-L gene can be incorporated into expression vectors. For example, a DNA sequence for a signal peptide (secretory leader) may be fused in frame to the Fas-L sequence so that the Fas-L is initially translated as a fusion protein comprising the signal peptide. A signal peptide that is functional in the intended host cells enhances extracellular secretion of the Fas-L polypeptide. The signal peptide is cleaved from the Fas-L polypeptide upon secretion of Fas-L from the cell.

Suitable host cells for expression of Fas-L polypeptides include prokaryotes, yeast or higher eukaryotic cells. Appropriate cloning and expression vectors for use with bacterial, fungal, yeast, and mammalian cellular hosts are described, for example, in Pouwels et al. Cloning Vectors: A Laboratory Manual, Elsevier, New York, (1985). Cell-free translation systems could also be employed to produce Fas-L polypeptides using RNAs derived from DNA constructs disclosed herein.

Prokaryotes include gram negative or gram positive organisms, for example, *E. coli* or *Bacilli.* Suitable prokaryotic host cells for transformation include, for example, *E. coli, Bacillus subtilis, Salmonella typhimurium,* and various other species within the genera *Pseudomonas, Streptomyces,* and *Staphylococcus.* In a prokaryotic host cell, such as *E. coli,* a Fas-L polypeptide may include an N-terminal methionine residue to facilitate expression of the recombinant polypeptide in the prokaryotic host cell. The N-terminal Met may be cleaved from the expressed recombinant Fas-L polypeptide.

Expression vectors for use in prokaryotic host cells generally comprise one or more phenotypic selectable marker genes. A phenotypic selectable marker gene is, for example, a gene encoding a protein that confers antibiotic resistance or that supplies an autotrophic requirement. Examples of useful expression vectors for prokaryotic host cells include those derived from commercially available plasmids such as the cloning vector pBR322 (ATCC 37017). pBR322 contains genes for ampicillin and tetracycline resistance and thus provides simple means for identifying transformed cells. An appropriate promoter and a Fas-L DNA sequence are inserted into the pBR322 vector. Other commercially available vectors include, for example, pKK223-3 (Pharmacia Fine Chemicals, Uppsala, Sweden) and pGEM1 (Promega Biotec, Madison, WI, USA).

Promoter sequences commonly used for recombinant prokaryotic host cell expression vectors include β-lactamase (penicillinase), lactose promoter system (Chang et al., Nature 275:615, 1978; and Goeddel et al., Nature 281:544, 1979), tryptophan (trp) promoter system (Goeddel et al., Nucl. Acids Res. 8:4057, 1980; and EP-A-36776) and tac promoter (Maniatis, Molecular Cloning: A Laboratory Manual, Cold Spring Harbor Laboratory, p. 412, 1982). A particularly useful prokaryotic host cell expression system employs a phage λ P_{L} promoter and a cI857ts thermolabile repressor sequence. Plasmid vectors available from the American Type Culture Collection which incorporate derivatives of the λ P_{L} promoter include plasmid pHUB2 (resident in *E. coli* strain JMB9 (ATCC 37092)) and pPLc28 (resident in *E*. *coli* RR 1 (ATCC 53082)).

Fas-L alternatively may be expressed in yeast host cells, preferably from the *Saccharomyces* genus (e.g., *S. cerevisiae*). Other genera of yeast, such as *Pichia* or *Kluyveromyces,* may also be employed. Yeast vectors will often contain an origin of replication sequence from a 2µ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionein, 3-phosphoglycerate kinase (Hitzeman et al., J. Biol. Chem. 255:2073, 1980) or other glycolytic enzymes (Hess et al., J. Adv. Enzyme Reg. 7:149, 1968; and Holland et al., Biochem. 17:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EPA-73,657. Another alternative is the glucose-repressible ADH2 promoter described by Russell et al. (J. Biol. Chem. 258:2674, 1982) and Beier et a]. (Nature 300:724, 1982). Shuttle vectors replicable in both yeast and *E. coli* may be constructed by inserting DNA sequences from pBR322 for selection and replication in *E. coli* (Amp^{r} gene and origin of replication) into the above-described yeast vectors.

The yeast α-factor leader sequence may be employed to direct secretion of the Fas-L polypeptide. The α-factor leader sequence is often inserted between the promoter sequence and the structural gene sequence. *See,* e.g., Kurjan et al., Cell 30:933, 1982 and Bitter et al., Proc. Natl. Acad. Sci. USA 81:5330, 1984. Other leader sequences suitable for facilitating secretion of recombinant polypeptides from yeast hosts are known to those of skill in the art. A leader sequence may be modified near its 3' end to contain one or more restriction sites. This will facilitate fusion of the leader sequence to the structural gene.

Yeast transformation protocols are known to those of skill in the art. One such protocol is described by Hinnen et al., Proc. Natl. Acad. Sci. USA 75:1929, 1978. The Hinnen et al. protocol selects for Trp⁺ transformants in a selective medium, wherein the selective medium consists of 0.67% yeast nitrogen base, 0.5% casamino acids, 2% glucose, 10 µg/ml adenine and 20 µg/ml uracil.

Yeast host cells transformed by vectors containing ADH2 promoter sequence may be grown for inducing expression in a "rich" medium. An example of a rich medium is one consisting of 1% yeast extract, 2% peptone, and 1% glucose supplemented with 80 µg/ml adenine and 80 µg/ml uracil. Derepression of the ADH2 promoter occurs when glucose is exhausted from the medium.

Mammalian or insect host cell culture systems could also be employed to express recombinant Fas-L polypeptides. Bacculovirus systems for production of heterologous proteins in insect cells are reviewed by Luckow and Summers, Bio/Technology 6:47 (1988). Established cell lines of mammalian origin also may be employed. Examples of suitable mammalian host cell lines include the COS-7 line of monkey kidney cells (ATCC CRL 1651) (Gluzman et al., Cell 23:175, 1981), L cells, C127 cells, 3T3 cells (ATCC CCL 163), Chinese hamster ovary (CHO) cells, HeLa cells, and BHK (ATCC CRL 10) cell lines, and the CVI/EBNA cell line derived from the African green monkey kidney cell line CVI (ATCC CCL 70) as described by McMahan et al. (EMBO J. 10: 2821, 1991).

Transcriptional and translational control sequences for mammalian host cell expression vectors may be excised from viral genomes. Commonly used promoter sequences and enhancer sequences are derived from Polyoma virus, Adenovirus 2, Simian Virus 40 (SV40), and human cytomegalovirus. DNA sequences derived from the SV40 viral genome, for example, SV40 origin, early and late promoter, enhancer, splice, and polyadenylation sites may be used to provide other genetic elements for expression of a structural gene sequence in a mammalian host cell. Viral early and late promoters are particularly useful because both are easily obtained from a viral genome as a fragment which may also contain a viral origin of replication (Fiers et al., Nature 273:113, 1978). Smaller or larger SV40 fragments may also be used, provided the approximately 250 bp sequence extending from the *Hind* III site toward the *Bgl* I site located in the SV40 viral origin of replication site is included.

Exemplary expression vectors for use in mammalian host cells can be constructed as disclosed by Okayama and Berg (Mol. Cell. Biol. 3:280, 1983). A useful system for stable high level expression of mammalian cDNAs in C127 murine mammary epithelial cells can be constructed substantially as described by Cosman et al. (Mol. Immunol. 23:935, 1986). A useful high expression vector, PMLSV N1/N4, described by Cosman et al., Nature 312:768, 1984 has been deposited as ATCC 39890. Additional useful mammalian expression vectors are described in EP-A-0367566, and in PCT application WO 91/18982, incorporated by reference herein. The vectors may be derived from retroviruses. For expression of Fas-L, a type II protein lacking a native signal sequence, a heterologous signal sequence may be added, such as the signal sequence for interleukin-7 (IL-7) described in United States Patent 4,965,195, the signal sequence for interleukin-2 receptor described in Cosman et al., Nature 312:768 (1984); the interleukin-4 receptor signal peptide described in EP 367,566; the type I interleukin-1 receptor signal peptide described in U.S. Patent 4,968,607; and the type II interleukin-1 receptor signal peptide described in EP 460,846.

### Purified Fas-L Protein

The present invention provides purified human and murine Fas-L proteins, which may be produced by recombinant expression systems as described above or purified from naturally occurring cells. The desired degree of purity depends on the intended use of the protein. A relatively high degree of purity is desired when the protein is to be administered *in vivo,* for example. Advantageously, Fas-L polypeptides are purified such that no protein bands corresponding to other proteins are detectable upon analysis by SDS-polyacrylamide gel electrophoresis (SDS-PAGE). It will be recognized by one skilled in the pertinent field that multiple bands corresponding to Fas-L protein may be visualized by SDS-PAGE, due to differential glycosylation, differential post-translational processing, and the like, as discussed above. A preparation of Fas-L protein is considered to be purified as long as no bands corresponding to different (non-Fas-L) proteins are visualized. Most preferably the Fas-L is purified to substantial homogeneity, as indicated by a single protein band upon analysis by SDS-PAGE. The protein band may be visualized by silver staining or (if the protein is radiolabeled) autoradiography.

In one embodiment, the Fas-L protein is a human protein characterized by the N-terminal amino acid sequence MQQPFNYPYPQI (amino acids 1-12 of SEQ ID NO:2). Such protein is encoded by DNA characterized by the 5'-terminal sequence ATGCAGCAGCCCTTCAATTACCCATATCCCCAGATC (nucleotides 93-128 of SEQ ID NO:1).

One process for producing the Fas-L protein comprises culturing a host cell transformed with an expression vector comprising a DNA sequence that encodes Fas-L under conditions such that Fas-L is expressed. The Fas-L protein is then recovered from culture medium or cell extracts, depending upon the expression system employed. As the skilled artisan will recognize, procedures for purifying the recombinant Fas-L will vary according to such factors as the type of host cells employed and whether or not the Fas-L is secreted into the culture medium.

For example, when expression systems that secrete the recombinant protein are employed, the culture medium first may be concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred. Finally, one or more reversed-phase high performance liquid chromatography (RP-HPLC) steps employing hydrophobic RP-HPLC media, (e.g., silica gel having pendant methyl or other aliphatic groups) can be employed to further purify Fas-L. Some or all of the foregoing purification steps, in various combinations, can be employed to provide a substantially homogeneous recombinant protein.

It is also possible to utilize an affinity column comprising the ligand binding domain of Fas to affinity-purify expressed Fas-L polypeptides. Fas-L polypeptides can be removed from an affinity column in a high salt elution buffer and then dialyzed into a lower salt buffer for use. Alternatively, the affinity column may comprise an antibody that binds Fas-L. Example 3 describes a procedure for employing the Fas-L protein of the present invention to generate monoclonal antibodies directed against Fas-L.

Recombinant protein produced in bacterial culture may be isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps. Finally, RP-HPLC can be employed for final purification steps. Microbial cells can be disrupted by any convenient method, including freeze-thaw cycling, sonication, mechanical disruption, or use of cell lysing agents.

Transformed yeast host cells are preferably employed to express Fas-L as a secreted polypeptide. This simplifies purification. Secreted recombinant polypeptide from a yeast host cell fermentation can be purified by methods analogous to those disclosed by Urdal et al. (J. Chromatog. 296:171, 1984). Urdal et al. describe two sequential, reversed-phase HPLC steps for purification of recombinant human IL-2 on a preparative HPLC column.

### Antibodies

Antibodies that are immunoreactive with the Fas-L polypeptides disclosed herein are provided. Polyclonal and monoclonal antibodies may be prepared by conventional techniques. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, NY, (1988). Production of monoclonal antibodies that are immunoreactive with Fas-L is further illustrated in example 3 below.

The Fas-L proteins of the present invention or antigenic fragments thereof may be employed as immunogens to generate polyclonal or monoclonal antibodies specific to the Fas-L immunogens. In one embodiment, a soluble Fas-L polypeptide is the immunogen. Cells expressing recombinant Fas-L on the cell surface also may be employed as immunogens. As a further alternative, a fusion protein comprising a Fas-L polypeptide (e.g., a fusion protein comprising the extracellular domain of Fas-L and an antibody Fc region polypeptide) is the immunogen.

Antigen-binding fragments of such antibodies, which may be produced by conventional techniques, are also encompassed by the present invention. Examples of such fragments include, but are not limited to, Fab, F(ab'), and F(ab')₂ fragments. Antibody fragments and derivatives produced by genetic engineering techniques are also provided.

The monoclonal antibodies of the present invention include chimeric antibodies, e.g., humanized versions of murine monoclonal antibodies. Such humanized antibodies may be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment may comprise the antigen biding site of a murine monoclonal antibody and a variable region fragment (lacking the antigen-binding site) derived from a human antibody. Procedures for the production of chimeric and further engineered monoclonal antibodies include those described in Riechmann et al. (Nature 332:323, 1988), Liu et al. (PNAS 84:3439, 1987), Larrick et al. (Bio/Technology 7:934, 1989), and Winter and Harris (TIPS 14:139, May, 1993).

One embodiment of the present invention is directed to monoclonal antibodies that block the binding of Fas-L to cell surface Fas antigen. Monoclonal antibodies can be screened in conventional assays for the ability to block binding of Fas-L to cells bearing Fas antigen.

### Properties and Uses of Fas-L and Antibodies that Bind Fas-L

The Fas antigen has been reported to mediate apoptosis and is believed to play a role in clonal deletion of self-reactive T-cells. (Itoh et al., Cell 66:233, 1991; Watanabe-Fukunage et al., Nature 356:314, 1992). Mice carrying the *lpr* mutation do not produce functional Fas protein capable of transducing the apoptotic signal (Watanabe-Fukunage et al., Nature*, supra*). These mice develop lymphoproliferative disorders characterized by the accumulation of CD4-CD8- thymocytes in lymph nodes and the spleen, hypergammaglobulinaemia, autoantibody production, rheumatoid factor, arthritis, and glomerulonephritis. (Watanabe-Fukunage et al., Nature*, supra*). Alderson et al. (J. Exp. Med., 178: 2231, 1993) report that, in addition to a role in induction of apoptosis in certain transformed cell lines, the Fas protein may play an important role in the activation and proliferation of normal T cells.

Studies employing the Fas-L and anti-Fas-L antibodies of the present invention thus may provide further insight into the development of self tolerance by the immune system and the etiology of autoimmune diseases. Interaction of Fas with Fas-L and the mechanism of signal transduction may be investigated. The antibodies may be used to block the interaction of Fas with Fas-L. Fas-L and antibodies reactive therewith may be used to promote or inhibit, respectively, biological effects that are mediated by the binding of Fas-L to Fas. The Fas-L and antibodies thus may be used to regulate such biological effects, *in vitro* or *in vivo.*

The Fas-L DNA and protein of the present invention are useful as research tools in elucidating the etiology of autoimmune disorders. Such disorders include, but are not limited to, systemic lupus erythematosus (SLE), immunoblastive lymphadenopathy (IBL), angioimmunoblastic lymphadenopathy (AIL), lymphogranulomatosis X (LgX), rheumatoid arthritis, diabetes, multiple schlerosis, and allergies. The use of Fas-L in treating graft versus host disease also may be explored.

Fas-L may be employed to induce apoptosis in cells bearing the Fas antigen. The Fas-L thus is a research reagent useful in studies of the mechanisms by which apoptosis occurs.

The Fas-L of the present invention may be used in developing treatments for disorders mediated (directly or indirectly) by Fas-L. Alternatively, a therapeutically effective amount of purified Fas-L protein may be administered to a patient afflicted with a disorder caused by defective or insufficient amounts of Fas-L. As a further alternative, the Fas-L DNA sequences may be employed in developing a gene therapy approach to treating such disorders.

The present invention provides pharmaceutical compositions comprising purified Fas-L and a physiologically acceptable carrier, diluent, or excipient. Such carriers, excipients and diluents will be nontoxic to recipients at the dosages and concentrations employed. Such compositions may comprise buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients commonly employed in pharmaceutical compositions. Neutral buffered saline or saline mixed with conspecific serum albumin are exemplary appropriate diluents. The composition may be formulated as a lyophilizate using appropriate excipient solutions (e.g. sucrose) as diluents. Additional examples of suitable carriers and their formulations are described in Remington's Pharmaceutical Sciences. 16th ed., 1980, Mack Publishing Company. Appropriate dosages and the frequency of administration will depend, of course, on such factors as the nature and severity of the indication being treated, the desired response, the size and condition of the patient and so forth.

For therapeutic use purified proteins of the present invention are to be administered to a patient, preferably a human, for treatment in a manner appropriate to the indication. Thus, for example, the pharmaceutical compositions can be administered by bolus injection, continuous infusion, sustained release from implants, or other suitable technique.

The Fas-L protein employed in the pharmaceutical compositions preferably is purified such that the Fas-L protein is substantially free of other proteins of natural or endogenous origin, desirably containing less than about 1% by mass of protein contaminants residual of production processes. Such compositions, however, can contain other proteins added as stabilizers, carriers, excipients or co-therapeutics. The Fas-L protein preferably is purified to substantial homogeneity, i.e., is detectable as a single protein band when analyzed by SDS-polyacrylamide gel electrophoresis.

The Fas-L polypeptides of the present invention also may be employed in *in vitro* assays for detection of Fas or Fas-L or the interactions thereof. The Fas-L of the present invention can be used in a binding assay to detect cells expressing Fas. For example, Fas-L or an extracellular domain or a fragment thereof can be conjugated to a detectable moiety such as ¹²⁵I. Radiolabeling with ¹²⁵I can be performed by any of several standard methodologies that yield a functional ¹²⁵I-Fas-L molecule labeled to high specific activity. Alternatively, another detectable moiety such as an enzyme that can catalyze a colorometric or fluorometric reaction, biotin or avidin may be used. Cells to be tested for Fas expression can be contacted with labeled Fas-L. After incubation, unbound labeled Fas-L is removed and binding is measured using the detectable moiety.

The Fas ligand proteins disclosed herein also may be employed to measure the biological activity of Fas protein in terms of binding affinity for Fas-L. To illustrate, Fas-L may be employed in a binding affinity study to measure the biological activity of a Fas protein that has been stored at different temperatures, or produced in different cell types. The biological activity of a Fas protein thus can be ascertained before it is used in a research study, for example.

Fas-L proteins find use as reagents that may be employed by those conducting "quality assurance" studies. e.g., to monitor shelf life and stability of Fas protein under different conditions. Fas ligands may be used in determining whether biological activity is retained after modification of a Fas protein (e.g., chemical modification, truncation, mutation, etc.). The binding affinity of the modified Fas protein for a Fas-L is compared to that ot an unmodified Fas protein to detect any adverse impact of the modification on biological activity of Fas.

A different use of a Fas ligand is as a reagent in protein purification procedures. Fas-L or Fas-L/Fc fusion proteins may be attached to a solid support material by conventional techniques and used to purify Fas by affinity chromatography. Antibodies of the present invention may be used to study the effects of inhibiting the biological activity of Fas-L, to detect the presence of Fas-L *in vitro* or *in vivo,* and in affinity chromatography for purifying Fas-L, for example. Antibodies of the present invention also find use when inhibition of Fas-L-mediated apoptosis of Fas antigen-bearing cells is desired. Antibodies (preferably monoclonal antibodies) that block binding of endogenous Fas-L to the Fas antigen on the cells are employed for this purpose. In one embodiment, the antibodies bind to cell surface Fas-L and inhibit binding of the cell surface Fas-L to Fas antigen on target cells.

One embodiment of the invention is directed to modes of treating disorders mediated by the interaction of Fas-L and Fas, comprising providing a composition comprising a therapeutically effective amount of an antibody that inhibits binding of a Fas-L to Fas, and a suitable diluent or carrier, for the administration to a patient afflicted with such a disease. The antibody preferably is a monoclonal antibody. Diseases for which therapeutic treatment with Fas-L specific antibodies of the present invention may be beneficial include, but are not limited to, SLE, rheumatoid arthritis, lyme disease, idiopathic CD4⁺ T lymphocytopenia, and the effects of human immunodeficiency virus (HIV) infection.

Activated human T cells are induced to undergo programmed cell death (apoptosis) upon triggering through the CD3/T cell receptor complex, a process termed activated-induced cell death (AICD). AICD has been observed in T cells freshly isolated from HIV-infected, but not from uninfected, individuals (Groux et al., J Exp. Med., 175:331, 1992; Meyaard et al., Science, 257:217, 1992). Thus, apoptosis may play a role in the depletion of CD4⁺ T cells and the progression to AIDS in HIV infected individuals.

For Fas-L to play a role in T cell depletion in HIV infection, two criteria must be fulfilled. First, Fas-L must be expressed, and therefore T cells must be activated by exposure to antigen. Second, T cells must be "primed" to become susceptible to Fas mediated apoptosis. In HIV⁺ patients, such priming of T cells may come from crosslinking of CD4 by GP-120/anti-GP-120 complexes. Such complexes have been shown to prime normal CD4⁺ T cells to undergo AICD *in vitro* and to cause T cells to undergo apoptosis in mice that express a human CD4 transgene (Wang et al, Eur. J. lmmunol., 24:1553, 1994). In addition, CD4⁺T cells undergo apoptotic cell death in mice treated with antibody to CD4, but this phenomenon does not occur in Fas deficient LPR mice (Wang et al., Eur. J. Immunol., 24:1549, 1994). The AICD seen in activated normal human T cells (such as PL-1 cells) and in freshly isolated T cells from HIV⁺ individuals is qualitatively identical. Therapeutic intervention for HIV-infected individuals with an antibody that inhibits the interaction of Fas-L with Fas thus may be possible.

For therapeutic use, purified antibodies of the present invention are to be administered to a patient, such as a human, for treatment in a manner appropriate to the indication. Compositions comprising an antibody that binds Fas-L and a suitable diluent or carrier are provided herein. Diluents, carriers, and other components of suitable compositions are as described above. In one embodiment, a pharmaceutical composition comprises a therapeutically effective amount of a monoclonal antibody that inhibits binding of Fas-L to cell surface Fas antigen, and a suitable diluent or carrier.

### Nucleic Acid Fragments

The present invention further provides fragments of the Fas-L nucleotide sequences presented herein. In one embodiment, such fragments comprise at least about 14 consecutive nucleotides of the human Fas-L cDNA isolated in example 1 (and deposited as ATCC 69527). Such fragments may comprise at least 14 nucleotides of the coding region of the DNA sequence of SEQ ID NO: 1. DNA and RNA complements of said fragments are provided herein, along with both single-stranded and double-stranded forms of the Fas-L DNA.

Among the uses of such Fas-L nucleic acid fragments is use as a probe. As one example, a probe corresponding to the extracellular domain of Fas-L may be employed. The probes find use in detecting the presence of Fas-L nucleic acids in *in vitro* assays and in such procedures as Northern and Southern blots. Cell types expressing Fas-L can be identified as well. Such procedures are well known, and the skilled artisan can choose a probe of suitable length, depending on the particular intended application.

Other useful fragments of the Fas-L nucleic acids are antisense or sense oligonucleotides comprising a single-stranded nucleic acid sequence (either RNA or DNA) capable of binding to target Fas-L mRNA (sense) or Fas-L DNA (antisense) sequences. Antisense or sense oligonucleotides, according to the present invention, comprise a fragment of the coding region of Fas-L cDNA. Such a fragment generally comprises at least about 14 nucleotides, preferably from about 14 to about 30 nucleotides. The ability to create an antisense or a sense oligonucleotide, based upon a cDNA sequence for a given protein is described in, for example, Stein and Cohen, Cancer Res. 48:2659, 1988 and van der Krol et al., BioTechniques 6:958, 1988.

Binding of antisense or sense oligonucleotides to target nucleic acid sequences results in the formation of duplexes that block translation (RNA) or transcription (DNA) by one of several means, including enhanced degradation of the duplexes, premature termination of transcription or translation, or by other means. The antisense oligonucleotides thus may be used to block expression of Fas-L proteins. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences. Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oliginucleotide for the target nucleotide sequence. Antisense or sense oligonucleotides may be introduced into a cell containing the target nucleic acid sequence by any gene transfer method, including, for example, CaPO₄-mediated DNA transfection, electroporation, or other gene transfer vectors such as Epstein-Barr virus. Antisense or sense oligonucleotides are preferably introduced into a cell containing the target nucleic acid sequence by insertion of the antisense or sense oligonucleotide into a suitable retroviral vector, then contacting the cell with the retrovirus vector containing the inserted sequence, either *in vivo* or *ex vivo*. Suitable retroviral vectors include, but are not limited to, the murine retrovirus M-MuLV, N2 (a retrovirus derived from M-MuLV), or or the double copy vectors designated DCTSA, DCT5B and DCT5C (see PCT Application WO 90/13641). Alternatively, other promotor sequences may be used to express the oligonucleotide.

Sense or antisense oligonucleotides may also be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

The following examples are provided to illustrate particular embodiments and not to limit the scope of the invention.

### EXAMPLE 1: Isolation of Murine Fas-L DNA

A 180bp fragment of murine Fas-L DNA was isolated by polymerase chain reaction (PCR), as follows. The template in the PCR was cDNA prepared from RNA derived from murine peripheral blood lymphocytes (PBL) that had been stimulated for 18 hours with phorbol 12-myristate 13-acetate (PMA, Sigma Chemical Co.). The 5' and 3' primers were oligonucleotides based on the rat Fas-L DNA sequence of Suda et al. (Cell, 75:1169, 1993). The primers define a 180 bp fragment near the 3' end of the Fas-L DNA. The PCR was conducted by conventional procedures. See, for example, Saiki et al., Science 239:487 (1988); Recombinant DNA Methodology, Wu et al., eds., Academic Press, Inc., San Diego (1989), pp. 189-196; and PCR Protocols: A Guide to Methods and Applications, Innis et al., eds., Academic Press, Inc. (1990).

The 180bp murine Fas-L DNA fragment isolated and amplified by PCR comprised nucleotides 643-822 of the murine Fas-L DNA sequence presented in SEQ ID NO:4. This DNA fragment was labeled with ³²P for use as a probe. A random primed DNA labeling kit (Amersham, Arlington Heights, Illinois) was used to radiolabel the probe.

A full-length murine Fas-L DNA was isolated as follows. A murine hybridoma cell line designated SC9 was generated by fusion of the T-cell hybridoma cell line BW5147 with the ∝B 10.5 CTL line described by Lynch, D. and R. Miller (J. Exp. Med. 179:31, 1994). The ∝B 10.5 CTL cells were stimulated with PMA and ionomycin (cultured in 500 ng/ml PMA and 3 µg/ml ionomycin for two hours at 37°C) prior to fusion. The SC9 hybridoma cell line was selected for Fas-L expression. Briefly, cells resulting from the fusion were assayed for Fas-L expression, including an assay for binding to a soluble Fas/Fc fusion protein. SC9 was isolated by fluorescence activated cell sorting (FACS), based on the cells binding to Fas/Fc.

The SC9 cells were stimulated with PMA and ionomycin as above, and RNA was extracted therefrom. cDNA was prepared from the RNA by conventional techniques and inserted and packaged into phage vector λgt 10 (Gigapak^{®} Stratagene Cloning Systems, La Jolla, CA). The resulting cDNA library was screened using the 180 bp murine Fas-L DNA fragment prepared above as a probe.

A positive clone was isolated and the nucleotide sequence of the cDNA insert was determined. This murine Fas-L nucleotide sequence is presented in SEQ ID NO:4, and the amino acid sequence encoded thereby is presented in SEQ ID NO:5. The protein comprises a cytoplasmic domain (amino acids 1-78), a transmembrane region (amino acids 79-103), and an extracellular domain (amino acids 104-279).

### EXAMPLE 2: Isolation of Human Fas-L DNA

Human Fas-L cDNA was isolated from a cDNA library derived from stimulated human peripheral blood lymphocytes. The procedure was as follows.

Peripheral blood lymphocytes (PBL) were obtained from normal human volunteers and treated with 10 ng/ml of OKT3 (an anti-CD3 antibody), and 10 ng/ml of human IL-2 for six days. The PBL cells were washed and stimulated with 500 ng/ml ionomycin (Calbiochem) and 10 ng/ml PMA for four hours. Messenger RNA was isolated from the stimulated PBL cells. cDNA synthesized on the mRNA template was packaged into λgt10 phage vectors (Gigapak^{®} Stratagene Cloning Systems, La Jolla, CA) according to manufacturer's instructions. Recombinant phage were then plated on *E. coli* strain KW251 and screened using standard plaque hybridization techniques.

The ³²P-labeled 180bp murine Fas-L probe prepared in example 1 was hybridized to the recombinant DNA phage at 37°C overnight. Hybridization was followed by washing twice with 6X SSC at room temperature, then washing twice with 2X SSC/0.1% SDS at 55°C. Positive (hybridizing) plaques were visualized by autoradiography.

Two of the positive plaques were purified and the inserts were amplified by PCR. Oligonucleotides that flank the cloning site (the EcoRI site of λgt10 into which the cDNA had been inserted) were employed as primers for the PCR. The cDNA inserts of the recombinant phage from both positive plaques were about 2.0kb in length. The PCR products from one clone were digested with EcoRI, and the desired fragment (containing the cDNA insert) was ligated into an EcoRI-digested cloning vector pBLUESCRIPT^{®}SK (Stratagene Cloning Systems, La Jolla, CA). *E. coli* strain DH5α cells containing the resulting recombinant vector (designated human Fas-L/pBS) were deposited with the American Type Culture Collection, Rockville, Maryland, U.S.A., on January 5, 1994, and assigned accession no. ATCC 69527. The deposit was made under the terms of the Budapest Treaty.

The DNA sequence of this human Fas-L DNA is presented in SEQ ID NO: 1. The amino acid sequence encoded by the isolated DNA is presented in SEQ ID NO:2. This human Fas-L protein comprises an N-terminal cytoplasmic domain (amino acids 1-80), a transmembrane region (amino acids 81-105), and an extracellular domain (amino acids 106-281). As will be understood by the skilled artisan, the transmembrane region is identified in accordance with conventional criteria for identifying that type of hydrophobic domain. The exact boundaries of the transmembrane region may vary slightly (most likely by no more than five amino acids on either end) from those presented above. Computer programs useful for identifying such hydrophobic regions in proteins are available.

The human Fas-L coding region DNA sequence (nucleotides 93-938 of SEQ ID NO: 1) is 82.89% identical to the rat Fas-L coding region DNA sequence presented in Suda et al. (Cell, 75: 1169, 1993). The human Fas-L amino acid sequence of SEQ ID NO: 2 is 77.98% identical to that of rat Fas-L. The degree of homology between human and rat Fas-L was neither known nor predictable prior to isolation of the human Fas-L cDNA disclosed herein.

The human Fas-L cDNA was excised from pBLUESCRIPT^{®}SK by digestion with Sall and Not I. The desired fragment was isolated and ligated into a mammalian expression vector pDC409 which had been digested with Sall and Not I.

pDC409 is derived from an expression vector designated pDC406, and differs from pDC406 in that a Bgl II site outside the mcs has been deleted so that the mcs Bgl II site is unique. Two Pme 1 sites and one Srf 1 site have been added to the mcs, and three stop codons (TAG) have been positioned downstream of the mcs to function in all three reading frames. A T7 primer/promoter has been added to aid in the DNA sequencing process.

Plasmid pDC406, which has been described by McMahan et al. (EMBO J. 10:2821, 1991) and in PCT application WO 91/18982 (hereby incorporated by reference), is an expression vector for use in mammalian cells, but is also replicable in *E. coli* cells.

pDC406 contains origins of replication derived from SV40, Epstein-Barr virus and pBR322 and is a derivative of HAV-EO described by Dower et al., J. Immunol. 142:4314 (1989). pDC406 differs from HAV-EO by the deletion of an intron present in the adenovirus 2 tripartite leader sequence in HAV-EO. DNA inserted into a multiple cloning site (containing a number of restriction endonuclease cleavage sites) is transcribed and translated using regulatory elements derived from HIV and adenovirus. The vector contains a gene that confers ampicillin resistance.

The recombinant vector (pDC409 containing human Fas-L DNA) was transfected into the monkey kidney cell line CV-1/EBNA-1 (ATCC CRL 10478). The CV-1/EBNA cell line was derived by transfection of the CV-1 cell line (ATCC CCL 70) with a gene encoding Epstein-Barr virus nuclear antigen-1 (EBNA-1) that constitutively expresses EBNA-1 driven from the human CMV intermediate-early enhancer/promoter as described by McMahan et al., *supra.* The EBNA-1 gene allows for episomal replication of expression vectors, such as pDC409, that contain the EBV origin of replication.

CV1-EBNA-1 cells transformed with the recombinant vector are cultivated in roller bottles to permit express of the Fas-L protein. Other suitable mammalian expression vectors may be substituted for pDC409.

### EXAMPLE 3

This example illustrates the preparation of monoclonal antibodies to Fas-L. Fas-L is expressed in mammalian host cells such as COS-7 or CVI-EBNA cells and purified using Fas/Fc affinity chromatography as described herein. The Fas-L may be the human or murine Fas-L described herein, or an immunogenic fragment thereof, e.g., the extracellular domain thereof.

Purified Fas-L can be used to generate monoclonal antibodies against Fas-L using conventional techniques, for example, those techniques described in U.S. Patent 4,411,993. Briefly, mice are immunized with Fas-L as an immunogen emulsified in complete Freund's adjuvant, and injected in amounts ranging from 10-100 µg subcutaneously or intraperitoneally. Ten to twelve days later, the immunized animals are boosted with additional Fas-L emulsified in incomplete Freund's adjuvant. Mice are periodically boosted thereafter on a weekly to bi-weekly immunization schedule. Serum samples are periodically taken by retro-orbital bleeding or tail-tip excision for testing by dot blot assay or ELISA (Enzyme-Linked Immunosorbent Assay), for Fas-L antibodies.

Following detection of an appropriate antibody titer, positive animals are provided one last intravenous injection of Fas-L in saline. Three to four days later, the animals are sacrificed, spleen cells harvested, and spleen cells are fused to a murine myeloma cell line (e.g., NS1 or Ag 8.653). Fusions generate hybridoma cells, which are plated in multiple microtiter plates in a HAT (hypoxanthine, aminopterin and thymidine) selective medium to inhibit proliferation of non-fused cells, myeloma hybrids, and spleen cell hybrids.

The hybridoma cells are screened by ELISA for reactivity against purified Fas-L by adaptations of the techniques disclosed in Engvall et al., Immunochem. 8:871, 1971 and in U.S. Patent 4,703,004. Positive hybridoma cells can be injected intraperitoneally into syngeneic BALB/c mice to produce ascites containing high concentrations of anti-Fas-L monoclonal antibodies. Alternatively, hybridoma cells can be grown *in vitro* in flasks or roller bottles by various techniques. Monoclonal antibodies produced in mouse ascites can be purified by ammonium sulfate precipitation, followed by gel exclusion chromatography. Alternatively, affinity chromatography based upon binding of antibody to protein A or protein G can also be used, as can affinity chromatography based upon binding to Fas-L.

## Claims

1. An isolated DNA encoding a human Fas-L polypeptide, wherein said Fas-L polypeptide is capable of binding Fas and is **characterized by** an N-terminal amino acid sequence MQQPFNYPYPQI and wherein said Fas-L polypeptide comprises the amino acid sequence of residues 1-281 of SEQ ID NO:2.

2. An isolated DNA according to claim 1, wherein said DNA comprises the nucleotide sequence of nucleotides 93-938 of SEQ ID NO:1.

3. An isolated DNA according to claim 1, wherein said DNA comprises the coding region of the cDNA insert of the recombinant vector deposited in strain ATCC 69527.

4. An isolated DNA encoding a soluble human Fas-L polypeptide, wherein said Fas-L polypeptide comprises the amino acid sequence of residues 106-281 of SEQ ID NO:2.

5. An isolated DNA according to claim 4, wherein said DNA comprises the nucleotide sequence of nucleotides 408-938 of SEQ ID NO:1.

6. An expression vector comprising a DNA as defined in any one of claims 1 to 5.

7. A process for preparing a Fas-L polypeptide, comprising culturing a host cell transformed with a vector as defined in claim 6 under conditions promoting expression of Fas-L, and recovering the Fas-L polypeptide from the culture.

8. A purified human Fas-L protein **characterized by** an N-terminal amino acid sequence MQQPFNYPYPQI, wherein said Fas-L protein is capable of binding Fas, and wherein said protein comprises the amino acid sequence of residues 1-281 of SEQ ID NO:2.

9. A soluble human Fas-L protein comprising the amino acid sequence of residues 106-281 of SEQ ID NO:2.

10. A human Fas-L protein of claim 8 encoded by the cDNA insert of the recombinant vector deposited in strain ATCC 69527.

11. An oligomer of Fas-L polypeptides comprising a polypeptide of any one of claims 8 to 10.

12. An oligomer according to claim 11 in the form of dimer or trimer of the polypeptide of any one of claims 8 to 10.

13. An oligomer according to claim 11 or 12, which is formed by disulfide bonds between cysteine residues on different Fas-L polypeptides or expressed as fusion polymers with or without spacer amino acid linking groups.

14. An oligomer according to claim 11, wherein said oligomer is a dimer and is created by fusing Fas-L to the Fc region of an antibody.

15. An isolated nucleic acid molecule comprising at least about 14 nucleotides of SEQ ID NO: 1, or the DNA or RNA complement thereof.

16. A human Fas-L polypeptide according to any of claims 8 to 10 for use in medicine.

17. An antibody immunoreactive with human Fas-L according to any of claims 8 to 10 for use in medicine.

18. Use of a human Fas-L polypeptide according to any of claims 8 to 10 or DNA encoding a human Fas-L polypeptide according to any of claims 11 to 14 in the preparation of an agent for the treatment of an autoimmune disease, graft versus host disease, or a disease mediated by the interaction of Fas-L and Fas selected from systemic lupus erythematosus (SLE), rheumatoid arthritis, lyme disease, idiopathic CD4⁺ T lymphocytopenia or the effects of human immunodeficiency virus (HIV) infection.

19. The use according to claim 18 in which the autoimmune disease is systemic lupus erythematosus (SLE), immunoblastive lymphadenopathy (IBL), angioimmunoblastic lymphadenopathy (AIL), lymphogranulomatosis X (LgX), rheumatoid arthritis, diabetes, multiple sclerosis or allergies.

20. Use of an antibody that is immunoreactive with human Fas-L according to any of claims 8 to 10 and that inhibits binding of a Fas-L polypeptide to Fas in the preparation of an agent for the treatment of a disease mediated by the interaction of Fas-L and Fas selected from systemic lupus erythematosus (SLE), rheumatoid arthritis, lyme disease, idiopathic CD4⁺ T lymphocytopenia or the effects of human immunodeficiency virus (HIV) infection.

21. A pharmaceutical composition comprising a human Fas-L polypeptide according to any of claims 8 to 10, and optionally a pharmaceutically acceptable excipient, diluent or carrier.

22. A pharmaceutical composition comprising an antibody immunoreactive with a human Fas-L polypeptide according to any of claims 8 to 10, and optionally a pharmaceutically acceptable excipient, diluent or carrier.

## Patentansprüche

1. Isolierte DNA, die ein menschliches Fas-L-Polypeptid kodiert, wobei das Fas-L-Polypeptid Fas binden kann und durch eine N-terminale Aminosäuresequenz MQQPFNYPYPQI **gekennzeichnet** ist und wobei das Fas-L-Polypeptid die Aminosäuresequenz der Reste 1 - 281 der SEQ ID NO: 2 umfasst.

2. Isolierte DNA gemäß Anspruch 1, wobei die DNA die Nucleotidsequenz der Nucleotide 93 - 938 der SEQ ID NO: 1 umfasst.

3. Isolierte DNA gemäß Anspruch 1, wobei die DNA die kodierende Region des cDNA-Inserts des rekombinanten Vektors umfasst, der im Stamm ATCC 69527 hinterlegt ist.

4. Isolierte DNA, die ein lösliches menschliches Fas-L-Polypeptid kodiert, wobei das Fas-L-Polypeptid die Aminosäuresequenz der Reste 106 - 281 der SEQ ID NO: 2 umfasst.

5. Isolierte DNA gemäß Anspruch 4, wobei die DNA die Nucleotidsequenz der Nucleotide 408 - 938 der SEQ ID NO: 1 umfasst.

6. Expressionsvektor, umfassend eine wie in einem der Ansprüche 1 bis 5 definierte DNA.

7. Verfahren zum Herstellen eines Fas-L-Polypeptids, umfassend das Züchten einer Wirtszelle, die mit einem wie in Anspruch 6 definierten Vektor transformiert ist, unter Bedingungen, die eine Expression von Fas-L fördern, und das Gewinnen des Fas-L-Polypeptids aus der Kultur.

8. Gereinigtes menschliches Fas-L-Protein, das durch eine N-terminale Aminosäuresequenz MQQPFNYPYPQI **gekennzeichnet** ist, wobei das Fas-L-Protein Fas binden kann und wobei das Protein die Aminosäuresequenz der Reste 1 - 281 der SEQ ID NO: 2 umfasst.

9. Lösliches menschliches Fas-L-Protein, umfassend die Aminosäuresequenz der Reste 106 - 281 der SEQ ID NO: 2.

10. Menschliches Fas-L-Protein nach Anspruch 8, das durch das cONA-Insert des im Stamm ATCC 69527 hinterlegten rekombinanten Vektors kodiert wird.

11. Oligomer aus Fas-L-Polypeptiden, umfassend ein Polypeptid nach einem der Ansprüche 8 bis 10.

12. Oligomer gemäß Anspruch 11 in Form eines Dimers oder Trimers des Polypeptids nach einem der Ansprüche 8 bis 10.

13. Oligomer gemäß Anspruch 11 oder 12, das durch Disulfidbindungen zwischen Cysteinresten auf unterschiedlichen Fas-L-Polypeptiden gebildet oder als Fusionspolymere mit oder ohne Verknüpfungsgruppen aus Spacer-Aminosäuren exprimiert wird.

14. Oligomer gemäß Anspruch 11, wobei das Oligomer ein Dimer ist und durch Fusionieren von Fas-L an die Fc-Region eines Antikörpers erzeugt wird.

15. Isoliertes Nucleinsäuremolekül, umfassend mindestens etwa 14 Nucleotide der SEQ ID NO: 1, oder das DNA- oder RNA-Komplement davon.

16. Menschliches Fas-L-Polypeptid gemäß einem der Ansprüche 8 bis 10 zur Verwendung in der Medizin.

17. Antikörper, der mit menschlichem Fas-L gemäß einem der Ansprüche 8 bis 10 eine Immunreaktion eingehen kann, zur Verwendung in der Medizin.

18. Verwendung eines menschlichen Fas-L-Polypeptids gemäß einem der Ansprüche 8 bis 10 oder von DNA, die ein menschliches Fas-L-Polypeptid gemäß einem der Ansprüche 11 bis 14 kodiert, bei der Herstellung eines Mittels zur Behandlung einer Autoimmunerkrankung, der Transplantat-gegen-Wirt-Krankheit oder einer durch die Wechselwirkung von Fas-L und Fas vermittelten Erkrankung, ausgewählt aus systemischem Lupus erythematodes (SLE), rheumatoider Arthritis, der Lyme-Krankheit, idiopathischer CD4⁺-T-Lymphozytopenie oder den Wirkungen einer Infektion mit menschlichem Immundefizienzvirus (HIV).

19. Verwendung gemäß Anspruch 18, wobei es sich bei der Autoimmunerkrankung um systemischen Lupus erythematodes (SLE), immunoblastische Lymphadenopathie (IBL), angioimmunoblastische Lymphadenopathie (AIL), Lymphogranulomatose X (LgX), rheumatoide Arthritis, Diabetes, multiple Sklerose oder Allergien handelt.

20. Verwendung eines Antikörpers, der mit menschlichem Fas-L gemäß einem der Ansprüche 8 bis 10 eine Immunreaktion eingehen kann und der die Bindung eines Fas-L-Polypeptids an Fas hemmt, bei der Herstellung eines Mittels zur Behandlung einer durch die Wechselwirkung von Fas-L und Fas vermittelten Erkrankung, ausgewählt aus systemischem Lupus erythematodes (SLE), rheumatoider Arthritis, der Lyme-Krankheit, idiopathischer CD4⁺-T-Lymphozytopenie oder den Wirkungen einer Infektion mit menschlichem Immundefizienzvirus (HIV).

21. Pharmazeutische Zusammensetzung, umfassend ein menschliches Fas-L-Polypeptid gemäß einem der Ansprüche 8 bis 10 und gegebenenfalls einen pharmazeutisch verträglichen Exzipienten, ein pharmazeutisch verträgliches Verdünnungsmittel oder einen pharmazeutisch verträglichen Träger.

22. Pharmazeutische Zusammensetzung, umfassend einen Antikörper, der mit einem menschlichem Fas-L-Polypeptid gemäß einem der Ansprüche 8 bis 10 eine Immunreaktion eingehen kann, und gegebenenfalls einen pharmazeutisch verträglichen Exzipienten, ein pharmazeutisch verträgliches Verdünnungsmittel oder einen pharmazeutisch verträglichen Träger.

## Revendications

1. ADN isolé codant pour un polypeptide Fas-L humain, dans lequel ledit polypeptide Fas-L est capable de lier Fas et est **caractérisé par** une séquence d'acides aminés N-terminaux MQQPFNYPYPQI et dans lequel ledit polypeptide Fas-L comprend la séquence d'acides aminés des résidus 1 à 281 de SEQ ID NO : 2.

2. ADN isolé selon la revendication 1, dans lequel ledit ADN comprend la séquence nucléotidique des résidus 93 à 938 de SEQ ID NO : 1.

3. ADN isolé selon la revendication 1, dans lequel ledit ADN comprend la région codante de l'insert d'ADNc du vecteur recombinant déposé sous la souche ATCC 69527.

4. ADN isolé codant pour un polypeptide Fas-L humain soluble, dans lequel ledit polypeptide Fas-L comprend la séquence d'acides aminés des résidus 106 à 281 de SEQ ID NO : 2.

5. ADN isolé selon la revendication 4, dans lequel ledit ADN comprend la séquence nucléotidique des nucléotides 408 à 938 de SEQ ID NO : 1.

6. Vecteur d'expression comprenant un ADN tel que défini dans l'une quelconque des revendications 1 à 5.

7. Procédé de préparation d'un polypeptide Fas-L, comprenant la culture d'une cellule hôte transformée avec un vecteur tel que défini dans la revendication 6 dans des conditions favorisant l'expression de Fas-L, et la récupération du polypeptide Fas-L à partir de la culture.

8. Protéine Fas-L humaine purifiée **caractérisée par** une séquence d'acides aminés N-terminaux MQQPFNYPYPQI, dans laquelle ladite protéine Fas-L est capable de lier Fas, et dans laquelle ladite protéine comprend la séquence d'acides aminés des résidus 1 à 281 de SEQ ID NO : 2.

9. Protéine Fas-L humaine soluble comprenant la séquence d'acides aminés des résidus 106 à 281 de SEQ ID NO : 2.

10. Protéine Fas-L humaine selon la revendication 8 codée par l'insert d'ADNc du vecteur recombinant déposé sous la souche ATCC 69527.

11. Oligomère de polypeptides Fas-L comprenant un polypeptide selon l'une quelconque des revendications 8 à 10.

12. Oligomère selon la revendication 11 sous la forme de dimère ou de trimère du polypeptide selon l'une quelconque des revendications 8 à 10.

13. Oligomère selon la revendication 11 ou 12, qui est formé par des liaisons disulfure entre les résidus cystéine sur différents polypeptides Fas-L ou exprimé sous la forme de polymères de fusion avec ou sans groupes liant les acides aminés espaceurs.

14. Oligomère selon la revendication 11, dans lequel ledit oligomère est un dimère et est créé en fusionnant Fas-L à la région Fc d'un anticorps.

15. Molécule d'acide nucléique isolé comprenant au moins 14 nucléotides de SEQ ID NO : 1, ou le complémentaire d'ADN ou d'ARN de celui-ci.

16. Polypeptide Fas-L humain selon l'une quelconque des revendications 8 à 10 pour une utilisation en médecine.

17. Anticorps immunoréactif avec le Fas-L humain selon l'une quelconque des revendications 8 à 10 pour une utilisation en médecine.

18. Utilisation d'un polypeptide Fas-L humain selon l'une quelconque des revendications 8 à 10 ou d'ADN codant pour un polypeptide Fas-L humain selon l'une quelconque des revendications 11 à 14 pour la préparation d'un agent destiné au traitement d'une maladie auto-immune, de la maladie du greffon contre l'hôte, ou d'une maladie médiée par l'interaction de Fas-L et de Fas choisie parmi le lupus érythémateux systémique (LES), la polyarthrite rhumatoïde, la maladie de Lyme, la lymphocytopénie idiopathique T CD4⁺ ou les effets d'une infection au virus de l'immunodéficience humaine (VIH).

19. Utilisation selon la revendication 18, dans laquelle la maladie auto-immune est le lupus érythémateux systémique (LES), la lymphadénopathie immunoblastique (LIB), la lymphadénopathie angio-immunoblastique (LAI), la lymphogranulomatose X (LgX), la polyarthrite rhumatoïde, le diabète, la sclérose en plaques ou des allergies.

20. Utilisation d'un anticorps qui est immunoréactif avec le Fas-L humain selon l'une quelconque des revendications 8 à 10 et qui inhibe la liaison d'un polypeptide Fas-L à Fas pour la préparation d'un agent destiné au traitement d'une maladie médiée par l'interaction de Fas-L et de Fas choisie parmi le lupus érythémateux systémique (LES), la polyarthrite rhumatoïde, la maladie de Lyme, la lymphocytopénie idiopathique T CD4⁺ ou les effets d'une infection au virus de l'immunodéficience humaine (VIH).

21. Composition pharmaceutique comprenant un polypeptide Fas-L humain selon l'une quelconque des revendications 8 à 10, et éventuellement un excipient, un diluant ou un support pharmaceutiquement acceptable.

22. Composition pharmaceutique comprenant un anticorps immunoréactif avec un polypeptide Fas-L humain selon l'une quelconque des revendications 8 à 10, et éventuellement un excipient, un diluant ou un support pharmaceutiquement acceptable.


### SEQUENCE LISTING

(1) GENERAL INFORMATION:
   (i) APPLICANT:
      (A) NAME: Immunex Corporation
      (B) STREET: 51 University Street
      (C) CITY: Seattle
      (D) STATE: WA
      (E) COUNTRY: US
      (F) POSTAL CODE (ZIP): 98101
   (ii) TITLE OF INVENTION: Ligand That Binds Fas Antigen
   (iii) NUMBER OF SEQUENCES: 5
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. Version #1.30 (EPO)
   (v) CURRENT APPLICATION DATA:
      APPLICATION NUMBER: EP 95907381.8
   (vi) PRIOR APPLICATION DATA:
      (A) APPLICATION NUMBER: US 08/179.138
      (B) FILING DATE: 07-JAN-1994
(2) INFORMATION FOR SEQ ID NO: 1:
   (i) SEQUENCE CHARACTERISTICS:
      (A) LENGTH: 1841 base pairs
      (B) TYPE: nucleic acid
      (C) STRANDEDNESS: single
      (D) TOPOLOGY: linear
   (ii) MOLECULE TYPE: cDNA to mRNA
   (ix) FEATURE:
      (A) NAME/KEY: CDS
      (B) LOCATION:93..938
   (xi) SEQUENCE DESCRIPTION: SEQ ID NO: 1:
   GAATTCCGGG TCCCGTCCTT GACACCTCAG CCTCTACAGG ACTGAGAAGA AGTAAAACCG        60
   TTTGCTGGGG CTGGCCTGAC TCACCAGCTG CC ATG CAG CAG CCC TTC AAT TAC        113
                                       Met Gln Gln Pro Phe Asn Tyr
                                        1               5
   CCA TAT CCC CAG ATC TAC TGG GTG GAC AGC AGT GCC AGC TCT CCC TGG        161
   Pro Tyr Pro Gln Ile Tyr Trp Val Asp Ser Ser Ala Ser Ser Pro Trp
           10                  15                  20
   GCC CCT CCA GGC ACA GTT CTT CCC TGT CCA ACC TCT GTG CCC AGA AGG        209
   Ala Pro Pro Gly Thr Val Leu Pro Cys Pro Thr Ser Val Pro Arg Arg
       25                  30                  35
   CCT GGT CAA AGG AGG CCA CCA CCA CCA CCG CCA CCG CCA CCA CTA CCA        257
   Pro Gly Gln Arg Arg Pro Pro Pro Pro Pro Pro Pro Pro Pro Leu Pro
   40                  45                  50                  55
   CCT CCG CCG CCG CCG CCA CCA CTG CCT CCA CTA CCG CTG CCA CCC CTG      305
   Pro Pro Pro Pro Pro Pro Pro Leu Pro Pro Leu Pro Leu Pro Pro Leu
                   60                  65                  70
   AAG AAG AGA GGG AAC CAC AGC ACA GGC CTG TGT CTC CTT GTG ATG TTT      353
   Lys Lys Arg Gly Asn His Ser Thr Gly Leu Cys Leu Leu Val Met Phe
               75                  80                  85
   TTC ATG GTT CTG GTT GCC TTG GTA GGA TTG GGC CTG GGG ATG TTT CAG      401
   Phe Met Val Leu Val Ala Leu Val Gly Leu Gly Leu Gly Met Phe Gln
           90                  95                  100
   CTC TTC CAC CTA CAG AAG GAG CTG GCA GAA CTC CGA GAG TCT ACC AGC      449
   Leu Phe His Leu Gln Lys Glu Leu Ala Glu Leu Arg Glu Ser Thr Ser
       105                 110                 115
   CAG ATG CAC ACA GCA TCA TCT TTG GAG AAG CAA ATA GGC CAC CCC AGT      497
   Gln Met His Thr Ala Ser Ser Leu Glu Lys Gln Ile Gly His Pro Ser
   120                 125                 130                 135
   CCA CCC CCT GAA AAA AAG GAG CTG AGG AAA GTG GCC CAT TTA ACA GGC      545
   Pro Pro Pro Glu Lys Lys Glu Leu Arg Lys Val Ala His Leu Thr Gly
                   140                 145                 150
   AAG TCC AAC TCA AGG TCC ATG CCT CTG GAA TGG GAA GAC ACC TAT GGA      593
   Lys Ser Asn Ser Arg Ser Met Pro Leu Glu Trp Glu Asp Thr Tyr Gly
               155                 160                 165
   ATT GTC CTG CTT TCT GGA GTG AAG TAT AAG AAG GGT GGC CTT GTG ATC      641
   Ile Val Leu Leu Ser Gly Val Lys Tyr Lys Lys Gly Gly Leu Val Ile
           170                 175                 180
   AAT GAA ACT GGG CTG TAC TTT GTA TAT TCC AAA GTA TAC TTC CGG GGT      689
   Asn Glu Thr Gly Leu Tyr Phe Val Tyr Ser Lys Val Tyr Phe Arg Gly
       185                 190                 195
   CAA TCT TGC AAC AAC CTG CCC CTG AGC CAC AAG GTC TAC ATG AGG AAC      737
   Gln Ser Cys Asn Asn Leu Pro Leu Ser His Lys Val Tyr Met Arg Asn
   200                 205                 210                 215
   TCT AAG TAT CCC CAG GAT CTG GTG ATG ATG GAG GGG AAG ATG ATG AGC      785
   Ser Lys Tyr Pro Gln Asp Leu Val Met Met Glu Gly Lys Met Met Ser
                   220                 225                 230
   TAC TGC ACT ACT GGG CAG ATG TGG GCC CGC AGC AGC TAC CTG GGG GCA      833
   Tyr Cys Thr Thr Gly Gln Met Trp Ala Arg Ser Ser Tyr Leu Gly Ala
               235                 240                 245
   GTG TTC AAT CTT ACC AGT GCT GAT CAT TTA TAT GTC AAC GTA TCT GAG      881
   Val Phe Asn Leu Thr Ser Ala Asp His Leu Tyr Val Asn Val Ser Glu
           250                 255                 260
   CTC TCT CTG GTC AAT TTT GAG GAA TCT CAG ACG TTT TTC GGC TTA TAT      929
   Leu Ser Leu Val Asn Phe Glu Glu Ser Gln Thr Phe Phe Gly Leu Tyr
       265                 270                 275
   AAG CTC TAA GAGAAGCACT TTGGGATTCT TTCCATTATG ATTCTTTGTT              978
   Lys Leu
   280
   ACAGGCACCG AGAATGTTGT ATTCAGTGAG GGTCTTCTTA CATGCATTTG AGGTCAAGTA   1038
   AGAAGACATG AACCAAGTGG ACCTTGAGAC CACAGGGTTC AAAATGTCTG TAGCTCCTCA   1098
   ACTCACCTAA TGTTTATGAG CCAGACAAAT GGAGGAATAT GACGGAAGAA CATAGAACTC   1158
   TGGGCTGCCA TGTGAAGAGG GAGAAGCATG AAAAAGCAGC TACCAGGTGT TCTACACTCA   1218
   TCTTAGTGCC TGAGAGTATT TAGGCAGATT GAAAAGGACA CCTTTTAACT CACCTCTCAA   1278
   GGTGGGCCTT GCTACCTCAA GGGGGACTGT CTTTCAGATA CATGGTTGTG ACCTGAGGAT   1338
   TTAAGGGATG GAAAAGGAAG ACTAGAGGCT TGCATAATAA GCTAAAGAGG CTGAAAGAGG   1398
   CCAATGCCCC ACTGGCAGCA TCTTCACTTC TAAATGCATA TCCTGAGCCA TCGGTGAAAC   1458
   TAACAGATAA GCAAGAGAGA TGTTTTGGGG ACTCATTTCA TTCCTAACAC AGCATGTGTA   1518
   TTTCCAGTGC AATTGTAGGG GTGTGTGTGT GTGTGTGTGT GTGTGTGTGT GTATGACTAA   1578
   AGAGAGAATG TAGATATTGT GAAGTACATA TTAGGAAAAT ATGGGTTGCA TTTGGTCAAG   1638
   ATTTTGAATG CTTCCTGACA ATCAACTCTA ATAGTGCTTA AAAATCATTG ATTGTCAGCT   1698
   ACTAATGATG TTTTCCTATA ATATAATAAA TATTTATGTA GATGTGCATT TTTGTGAAAT   1758
   GAAAACATGT AATAAAAAGT ATATGTTAGG ATACAAATAA AAAAAAAAAA AAAAAAAAAA   1818
   AAAAAAAAAA AAAACCGGAA TTC                                           1841
(2) INFORMATION FOR SEQ ID NO: 2:
   (i) SEQUENCE CHARACTERISTICS:
      (A) LENGTH: 281 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Gln Gln Pro Phe Asn Tyr Pro Tyr Pro Gln Ile Tyr Trp Val Asp
     1               5                  10                  15
   Ser Ser Ala Ser Ser Pro Trp Ala Pro Pro Gly Thr Val Leu Pro Cys
                20                  25                  30
   Pro Thr Ser Val Pro Arg Arg Pro Gly Gln Arg Arg Pro Pro Pro Pro
            35                  40                  45
   Pro Pro Pro Pro Pro Leu Pro Pro Pro Pro Pro Pro Pro Pro Leu Pro
        50                  55                  60
   Pro Leu Pro Leu Pro Pro Leu Lys Lys Arg Gly Asn His Ser Thr Gly
    65                  70                  75                  80
   Leu Cys Leu Leu Val Met Phe Phe Met Val Leu Val Ala Leu Val Gly
                    85                  90                  95
   Leu Gly Leu Gly Met Phe Gln Leu Phe His Leu Gln Lys Glu Leu Ala
               100                 105                 110
   Glu Leu Arg Glu Ser Thr Ser Gln Met His Thr Ala Ser Ser Leu Glu
           115                 120                 125
   Lys Gln Ile Gly His Pro Ser Pro Pro Pro Glu Lys Lys Glu Leu Arg
       130                 135                 140
   Lys Val Ala His Leu Thr Gly Lys Ser Asn Ser Arg Ser Met Pro Leu
   145                 150                 155                 160
   Glu Trp Glu Asp Thr Tyr Gly Ile Val Leu Leu Ser Gly Val Lys Tyr
                   165                 170                 175
   Lys Lys Gly Gly Leu Val Ile Asn Glu Thr Gly Leu Tyr Phe Val Tyr
               180                 185                 190
   Ser Lys Val Tyr Phe Arg Gly Gln Ser Cys Asn Asn Leu Pro Leu Ser
           195                 200                 205
   His Lys Val Tyr Met Arg Asn Ser Lys Tyr Pro Gln Asp Leu Val Met
       210                 215                 220
   Met Glu Gly Lys Met Met Ser Tyr Cys Thr Thr Gly Gln Met Trp Ala
   225                 230                 235                 240
   Arg Ser Ser Tyr Leu Gly Ala Val Phe Asn Leu Thr Ser Ala Asp His
                   245                 250                 255
   Leu Tyr Val Asn Val Ser Glu Leu Ser Leu Val Asn Phe Glu Glu Ser
               260                 265                 270
   Gln Thr Phe Phe Gly Leu Tyr Lys Leu
           275                 280
(2) INFORMATION FOR SEQ ID NO: 3:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 3:
   Asp Tyr Lys Asp Asp Asp Asp Lys
   1               5
(2) INFORMATION FOR SEQ ID NO: 4:
   (i) SEQUENCE CHARACTERISTICS:
      (A) LENGTH: 1171 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:31..870
   (xi) SEQUENCE DESCRIPTION: SEQ 10 NO: 4:
   GAAGGAAACC CTTTCCTGGG GCTGGGTGCC ATG CAG CAG CCC ATG AAT TAC CCA       54
                                    Met Gln Gln Pro Met Asn Tyr Pro
                                     1               5
   TGT CCC CAG ATC TTC TGG GTA GAC AGC AGT GCC ACT TCA TCT TGG GCT       102
   Cys Pro Gln Ile Phe Trp Val Asp Ser Ser Ala Thr Ser Ser Trp Ala
        10                  15                  20
   CCT CCA GGG TCA GTT TTT CCC TGT CCA TCT TGT GGG CCT AGA GGG CCG       150
   Pro Pro Gly Ser Val Phe Pro Cys Pro Ser Cys Gly Pro Arg Gly Pro
    25                  30                  35                  40
   GAC CAA AGG AGA CCG CCA CCT CCA CCA CCA CCT GTG TCA CCA CTA CCA       198
   Asp Gln Arg Arg Pro Pro Pro Pro Pro Pro Pro Val Ser Pro Leu Pro
                    45                  50                  55
   CCG CCA TCA CAA CCA CTC CCA CTG TCG CCA CTG ACC CCT CTA AAG AAG       246
   Pro Pro Ser Gln Pro Leu Pro Leu Ser Pro Leu Thr Pro Leu Lys Lys
                60                  65                  70
   AAG GAC CAC AAC ACA AAT CTG TGG CTA CCG GTG GTA TTT TTC ATG GTT       294
   Lys Asp His Asn Thr Asn Leu Trp Leu Pro Val Val Phe Phe Met Val
            75                  80                  85
   CTG GTG GGT CTG GTT GGA ATG GGA TTA GGA ATG TAT CAG CTC TTC CAC       342
   Leu Val Gly Leu Val Gly Met Gly Leu Gly Met Tyr Gln Leu Phe His
        90                  95                 100
   CTG CAG AAG GAA CTG GCA GAA CTC CGT GAG TTC ACC AAC CAA AGC CTT       390
   Leu Gln Lys Glu Leu Ala Glu Leu Arg Glu Phe Thr Asn Gln Ser Leu
   105                 110                 115                 120
   AAA GTA TCA TCT TTT GAA AAG CAA ATA GCC AAC CCC AGT ACA CCC TCT       438
   Lys Val Ser Ser Phe Glu Lys Gln Ile Ala Asn Pro Ser Thr Pro Ser
                   125                 130                 135
   GAA AAA AAA GAG CCG AGG AGT GTG GCC CAT TTA ACA GGG AAC CCC CAC       486
   Glu Lys Lys Glu Pro Arg Ser Val Ala His Leu Thr Gly Asn Pro His
               140                 145                 150
   TCA AGG TCC ATC CCT CTG GAA TGG GAA GAC ACA TAT GGA ACC GCT CTG       534
   Ser Arg Ser Ile Pro Leu Glu Trp Glu Asp Thr Tyr Gly Thr Ala Leu
           155                 160                 165
   ATC TCT GGA GTG AAG TAT AAG AAA GGT GGC CTT GTG ATC AAC GAA ACT       582
   Ile Ser Gly Val Lys Tyr Lys Lys Gly Gly Leu Val Ile Asn Glu Thr
       170                 175                 180
   GGG TTG TAC TTC GTG TAT TCC AAA GTA TAC TTC CGG GGT CAG TCT TGC       630
   Gly Leu Tyr Phe Val Tyr Ser Lys Val Tyr Phe Arg Gly Gln Ser Cys
   185                 190                 195                 200
   AAC AAC CAG CCC CTA AAC CAC AAG GTC TAT ATG AGG AAC TCT AAG TAT       678
   Asn Asn Gln Pro Leu Asn His Lys Val Tyr Met Arg Asn Ser Lys Tyr
                   205                 210                 215
   CCT GAG GAT CTG GTG CTA ATG GAG GAG AAG AGG TTG AAC TAC TTC ACT       726
   Pro Glu Asp Leu Val Leu Met Glu Glu Lys Arg Leu Asn Tyr Phe Thr
               220                 225                 230
   ACT GGA CAG ATA TGG GCC CAC AGC AGC TAC CTG GGG GCA GTA TTC AAT       774
   Thr Gly Gln Ile Trp Ala His Ser Ser Tyr Leu Gly Ala Val Phe Asn
           235                 240                 245
   CTT ACC AGT GCT GAC CAT TTA TAT GTC AAC ATA TCT CAA CTC TCT CTG      822
   Leu Thr Ser Ala Asp His Leu Tyr Val Asn Ile Ser Gln Leu Ser Leu
       250                 255                 260
   ATC AAT TTT GAG GAA TCT AAG ACC TTT TTC GGC TTG TAT AAG CTT TAA      870
   Ile Asn Phe Glu Glu Ser Lys Thr Phe Phe Gly Leu Tyr Lys Leu
   265                 270                 275                 280
   AAGAAAAAGC ATTTTAAAAT GATCTACTAT TCTTTATCAT GGGCACCAGG AATATTGTCT    930
   TGAATGAGAG TCTTCTTAAG ACCTATTGAG ATTAATTAAG ACTACATGAG CCACAAAGAC    990
   CTCATGACCG CAAGGTCCAA CAGGTCAGCT ATTTCATTTT CTCGAGGTCC ATGGAGTGGT   1050
   CCTTAATGCC TGCATCATGA GCCAGATGGA AGGAGGTCTG TGACTGAGGG GACATAAAGC   1110
   TTGGGGCTGC TGTGTGACAA TGCAGAGGCA CAGAGGAAAG AACTGTCTGA TGTTAAATGG   1170
   C                                                                   1171
(2) INFORMATION FOR SEQ ID NO: 5:
   (i) SEQUENCE CHARACTERISTICS:
      (A) LENGTH: 279 amino acids
      (B) TYPE: amino acid
      (D) TOPOLOGY: linear
   (ii) MOLECULE TYPE: protein
   (xi) SEQUENCE DESCRIPTION: SEQ ID NO: 5:
   Met Gln Gln Pro Met Asn Tyr Pro Cys Pro Gln Ile Phe Trp Val Asp
   1               5                   10                  15
   Ser Ser Ala Thr Ser Ser Trp Ala Pro Pro Gly Ser Val Phe Pro Cys
               20                  25                  30
   Pro Ser Cys Gly Pro Arg Gly Pro Asp Gln Arg Arg Pro Pro Pro Pro
           35                  40                  45
   Pro Pro Pro Val Ser Pro Leu Pro Pro Pro Ser Gln Pro Leu Pro Leu
       50                  55                  60
   Ser Pro Leu Thr Pro Leu Lys Lys Lys Asp His Asn Thr Asn Leu Trp
   65                  70                  75                  80
   Leu Pro Val Val Phe Phe Met Val Leu Val Gly Leu Val Gly Met Gly
                   85                  90                  95
   Leu Gly Met Tyr Gln Leu Phe His Leu Gln Lys Glu Leu Ala Glu Leu
               100                 105                 110
   Arg Glu Phe Thr Asn Gln Ser Leu Lys Val Ser Ser Phe Glu Lys Gln
           115                 120                 125
   Ile Ala Asn Pro Ser Thr Pro Ser Glu Lys Lys Glu Pro Arg Ser Val
       130                 135                 140
   Ala His Leu Thr Gly Asn Pro His Ser Arg Ser Ile Pro Leu Glu Trp
   145                 150                 155                 160
   Glu Asp Thr Tyr Gly Thr Ala Leu Ile Ser Gly Val Lys Tyr Lys Lys
                   165                 170                 175
   Gly Gly Leu Val Ile Asn Glu Thr Gly Leu Tyr Phe Val Tyr Ser Lys
               180                 185                 190
   Val Tyr Phe Arg Gly Gln Ser Cys Asn Asn Gln Pro Leu Asn His Lys
           195                 200                 205
   Val Tyr Met Arg Asn Ser Lys Tyr Pro Glu Asp Leu Val Leu Met Glu
       210                 215                 220
   Glu Lys Arg Leu Asn Tyr Phe Thr Thr Gly Gln Ile Trp Ala His Ser
   225                 230                 235                 240
   Ser Tyr Leu Gly Ala Val Phe Asn Leu Thr Ser Ala Asp His Leu Tyr
                   245                 250                 255
   Val Asn Ile Ser Gln Leu Ser Leu Ile Asn Phe Glu Glu Ser Lys Thr
               260                 265                 270
   Phe Phe Gly Leu Tyr Lys Leu
           275